(19) Europäisches Patentamt
European Patent Office
Office européen des brevets

(11) EP 0 740 143 B1

# (12) EUROPÄISCHE PATENTSCHRIFT

(45) Veröffentlichungstag und Bekanntmachung des Hinweises auf die Patenterteilung:
**02.01.2003 Patentblatt 2003/01**

(51) Int Cl.$^7$: **G01N 11/16**

(21) Anmeldenummer: **96104705.7**

(22) Anmeldetag: **25.03.1996**

(54) **Schwingquarzsensor**

Quartz oscillator sensor

Détecteur à cristal oscillateur

(84) Benannte Vertragsstaaten:
**DE FR GB**

(30) Priorität: **05.04.1995 DE 19512739**

(43) Veröffentlichungstag der Anmeldung:
**30.10.1996 Patentblatt 1996/44**

(73) Patentinhaber: **Ticona GmbH**
**65451 Kelsterbach (DE)**

(72) Erfinder:
- **Schönfeld, Axel, Dr.**
  **65207 Wiesbaden (DE)**
- **Feucht, Gernot, Dr.**
  **67112 Mutterstadt (DE)**
- **Frank, Georg, Dr.**
  **72074 Tübingen (DE)**

(56) Entgegenhaltungen:
**WO-A-91/05998** **US-A- 3 778 229**
**US-A- 4 243 631** **US-A- 5 185 129**

- **ANALYTICAL CHEMISTRY, Band 57, Nr. 3, 1985 H.M. FOG "Piezolectric Crystal Detector for the Monitoring of Ozone in Working Environments" Seiten 2634-2638**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die Erfindung beschreibt einen Sensor auf der Basis des piezoelektrischen Effektes, wobei der Sensor mit einer aktiven, massenwirksamen Schicht aus Polymeren, die olefinische Seitengruppen enthalten, beschichtet ist, sowie seine Verwendung zum Nachweis von Ozon.

Es ist bekannt (Analyt. Chem., 57(13), 2634-8, 1985), daß Ozon mit einem piezoelektrischen, mit 1,4-Polybutadien beschichteten Sensor nachgewiesen werden kann. Problematisch am hier vorgestellten Verfahren sind die Herstellung des Polymerüberzugs durch Auftragen mit einer Bürste. Dabei kann die Oberfläche der Kontaktierung verletzt werden. Die Homogenität der Schicht kann durch dieses Verfahren ebenfalls nicht reproduzierbar sein, was durch den angegebenen Frequenzänderungsbereich (2 000 bis 10 000 Hz) bestätigt wird. Weiter sind die beobachteten Frequenzänderungen durch die beaufschlagten Ozonmengen im ppb-Bereich so klein, daß sie in der Größenordnung des Rauschens der Eigenfrequenz des Piezokristalls liegen (3 bis 30 Hz).

Bei der Reaktion des Ozons mit dem 1,4-Polybutadien entstehen niedermolekulare Verbindungen, die sich partiell verflüchtigen können. Dies führt zu einer entgegengesetzten Massenänderung und somit zu einem Fehler in der Konzentrationsbestimmung des Gases. Ferner zeigen niedermolekulare Verbindungen, insbesondere in dünnen Schichten, eine starke Kristallisationsneigung, die die Schwingfähigkeit des Quarzes in der Regel beeinträchtigt.

Polybutadien neigt im allgemeinen schon bei leicht erhöhten Temperaturen zu starker Vernetzungsreaktion, so daß ein solcher Schwingquarz-Sensor auch in Ozon-freier Luft schnell altert und unbrauchbar wird.

Es ist weiterhin bekannt, daß elektronische Frequenzgeneratoren zur Schwingungserzeugung ein piezoelektrisches Element aus Quarz oder PZT-Keramik nutzen. Eine der Resonanzfrequenzen wird zur Detektion von Massenänderungen ausgewählt und vom zugeschalteten externen Frequenzgenerator verstärkt, wobei es sich im Frequenzbereich bis ca. 20 MHz um die Grundschwingung handelt, die dabei resonant angeregt wird.

Bei Piezoelektrika gilt für die Frequenzänderung Δf die folgende Funktion (Sauerbrey-Gleichung):

$$\Delta f = -2{,}3 * 10^6 * F^2 * \Delta m/A$$

wobei A die schwingende Fläche, F die Grundschwingung und Δm die Massenänderung darstellt.
Wird eine schwingende Fläche (z.B. eine Quarzscheibe) mit einem Überzug versehen, so ändert sich die Frequenz des Sensor-Systems aufgrund der Massenzunahme.

Hat der Überzug absorbierende Eigenschaften bezüglich einer oder mehrerer Stoffe im Umgebungsmedium, reagiert das schwingende System mit einer Frequenzänderung auf eine stattfindende Absorption. Die Eigenschaften des Sensors (Selektivität, Empfindlichkeit, Regenerierbarkeit, Kumulierbarkeit) können durch entsprechende Auswahl des Absorbers in weiten Grenzen eingestellt werden.

Dabei ist jedoch zu beachten, daß die Schwingungseigenschaften der Piezoelektrika durch den Überzug nicht beeinträchtigt werden dürfen. Ferner darf der Absorber nicht unter Bildung flüchtiger Substanzen mit den zu detektierenden Stoffen reagieren. Für einen sinnvollen Einsatz ist weiterhin eine schnelle Umsetzung mit dem zu detektierenden Material notwendig.

Die Schwingungsfähigkeit des Piezokristalls geht im allgemeinen verloren, wenn der aufgebrachte Absorber auf dem Piezokristall kristalliner oder teilkristalliner Natur ist. Eine Vorhersage ist aber in keinem Fall möglich. Auch bei der Verwendung von organischen Substanzen können die benötigten Eigenschaften im allgemeinen nicht zuverlässig exakt eingestellt werden. Die Auswahl der einzusetzenden Substanz ist daher mehr oder weniger empirisch.

Aufgabe der Erfindung ist es, die genannten Nachteile zu umgehen und einfache, zuverlässige Methoden hinsichtlich der Herstellung der Piezoelektrika und des Nachweises von Ozon bereitzustellen.

Die Erfindung betrifft daher einen Sensor aus einem piezoelektrischen Kristall, der mit einer ein olefinisches Seitengruppenpolymer enthaltenden Beschichtung versehen ist.

Durch den Einsatz von Polymeren, die olefinische Seitengruppen enthalten, auf Piezokristallen mit geeigneter Beschichtungs- und Nachbehandlungstechnik ist es gelungen, die Nachteile auszuschalten und die gewünschten Eigenschaften, z.B. hohe Auflösung und Selektivität, sowie gute Alterungsbeständigkeit zu erhalten, wodurch Detektoren zum quantitativen Nachweis von Ozon zur Verfügung gestellt werden.

Geeignet sind Polymere, die olefinische Seitengruppen enthalten oder Mischungen daraus. Bei diesen Verbindungen reagiert die olefinische Doppelbindung mit dem Ozon unter Bildung von Ozoniden. Die Struktur der Hauptkette zeigt hier keinen Einfluß.Es können beispielsweise substituierte Polyacrylate, substituierte Polystyrole oder substituierte Polyester eingesetzt werden. Die Verbindungen müssen olefinische Doppelbindungen in der Seitengruppe aufweisen. Bei Verwendung von Copolymeren ist es im allgemeinen zweckmäßig, daß mindestens 3 % olefinische Seitengruppen, bevorzugt mindestens 15 % olefinische Seitengruppen vorhanden sind.

Im allgemeinen sind Polymere geeignet, die ein mittleres Molekulargewicht von 2 000 bis 2 000 000, vorzugsweise von 10 000 bis 500 000, insbesondere 10 000 bis 100 000, bestimmt durch GPC, aufweisen.

Gemäß der Erfindung können solche Kristalle von anorganischen Substanzen verwendet werden, die den piezoelektrischen Effekt zeigen.

Bevorzugt sind Erdalkalititanate, Blei/Zirkoniumtitanate und Quarze, insbesondere Bariumtitanat und Quarz im AT-Schnitt, bei denen die piezoelektrischen Eigenschaften eine besonders geringe Temperaturabhängigkeit aufweisen.

Im allgemeinen weisen die eingesetzten Piezokristalle eine Grundschwingung in einem Frequenzbereich von 20 kHz bis 100 MHz, bevorzugt von 0,1 MHz bis 50 MHz und insbesondere von 0,1 MHz bis 30 MHz auf.

Das eingesetze Polymer oder die Polymermischung kann über allgemeine Beschichtungsverfahren auf die Piezokristalle einseitig oder beidseitig aufgebracht werden. Bevorzugt sind dabei Beschichtungsverfahren, die auf Polymer- oder Monomerlösungen basieren, z.B. Spincoating, Dipcoating oder Sprühverfahen. Dabei sind alle organischen Substanzen geeignet, die das jeweilige Polymer oder Monomer in einem definierten Temperaturintervall lösen, beispielsweise Chloroform. Bei der Verwendung von Monomerlösung kann die Polymerisation durch allgemeine Oberflächenpolymerisationstechniken wie Laserinduktion oder Temperaturerhöhung durchgeführt werden.

Gemäß der Erfindung erfolgt die Nachbehandlung der aufgebrachten Polymerschicht durch Trocknung in handelsüblichen Trocknungsanlagen an Luft in Schutzgas oder unter verminderten Druck bei Temperaturen von 0 bis 350°C, bevorzugt 30 bis 300°C und insbesondere von 50 bis 300°C.
Es ist auch möglich, zur Erzielung dickerer Polymerschichten mehrere Belegungs- und Trocknungsschritte iterativ zu wiederholen.

Die Belegung des verwendeten Piezokristalls nach der Trocknung liegt bei 1 $ng/cm^2$ bis 1 $g/cm^2$, bevorzugt 5 $ng/cm^2$ bis 10 $mg/cm^2$ und insbesondere bei 10 $ng/cm^2$ bis 2 $mg/cm^2$.

Beispielsweise wird ein kontaktierter Schwingquarz (Grundschwingung zwischen 0,1 und 30 MHz) mittels Dipcoating, Spincoating oder Sprühverfahren mit in einem Lösungsmittel (z.B. Chloroform, Toluol) gelöstem Polymer beschichtet. Diese Beschichtung kann einseitig oder auf beiden Seiten der Quarzscheibe durch ein- oder mehrfache Wiederholung des Beschichtungsvorgangs erfolgen. Nach der Beschichtung wird der Sensor in einer üblichen Trocknungsanlage an Luft oder unter vermindertem Druck getrocknet.

Nach der Trocknung wird der Quarz auf Schwingfähigkeit kontrolliert. Die Absorberschicht-Masse kann aus der vorstehend beschriebenen Sauerbrey-Gleichung bestimmt werden.

Der so hergestellte Sensor wird in einer Durchflußzelle mit definiertem Volumenstrom dem zu prüfenden Gas ausgesetzt. Die Sensorfrequenz wird entweder direkt ausgewertet oder mit einer stabilisierten Referenzfrequenz gemischt und dann ausgewertet (Auftragung der Frequenz oder der Frequenzänderung gegen die Zeit). Die Signaländerung kann durch nachgeschaltete Prozessoren direkt in Massenänderungen umgerechnet und auf einer Anzeige sichtbar gemacht werden.

Beispiele:

Kommerzielle HC-18U Quarze (Grundfrequenz: 11,5 MHz) wurden aus ihrem Schutzgehäuse gelötet und in eine 1 %ige Lösung eines Polymers der Formel (I)

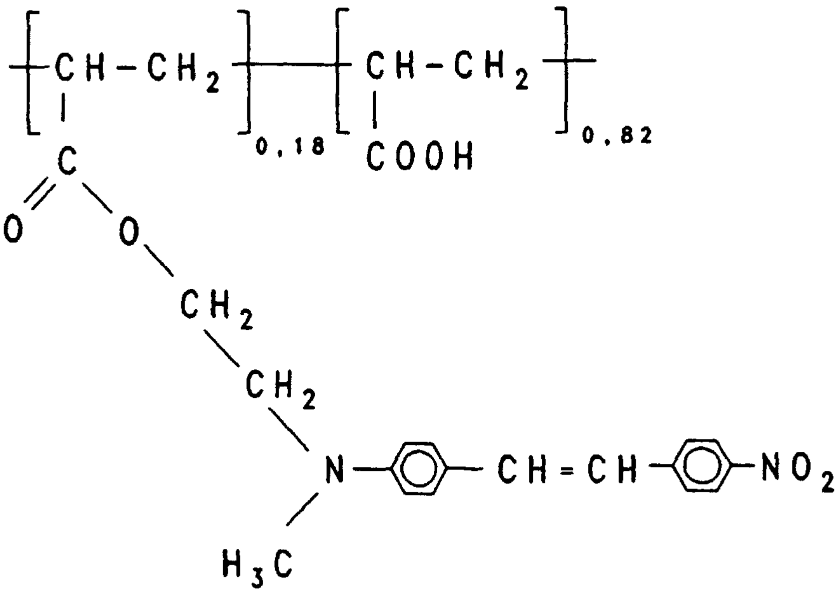

(I)

in Chloroform getaucht. Anschließend wurden die Sensoren bei 70 °C für 5 Stunden unter vermindertem Druck getrocknet. Die Schwingungsfähigkeit der beschichteten Sensoren wurde mit einem transistorisierten Oszillator, der Schwingquarze zwischen 0,1 und 30 MHz in Parallelresonanz schwingen läßt und einem 10 MHz Frequenzzähler (Auflösung 0,1 Hz) mit zuschaltbarem Vorteiler und thermostatisierter Torzeitbasis getestet.

Sensor 1:

Belegung mit (I): 3,78 mg
$O_3$ - Konzentration: 1 ppm
Strömungsgeschwindigkeit: ~ 100 l/h

| Zeit [h] | 0 | 4,5 | 5 | 5,5 | 6 | 6,5 | 7 | 7,5 | 8 | 8,5 |
|---|---|---|---|---|---|---|---|---|---|---|
| Δf [Hz] | 0 | 136 | 144 | 156 | 168 | 176 | 184 | 196 | 200 | 208 |

| Zeit [h] | 9 | 9,5 | 10 | 10,5 | 11 | 11,5 | 12 | 12,5 | 13 | 15 |
|---|---|---|---|---|---|---|---|---|---|---|
| Δf [Hz] | 216 | 224 | 236 | 240 | 248 | 252 | 260 | 264 | 276 | 296 |

Sensor 2:

Belegung mit (I): 3,89 mg
$O_3$ - Konzentration: 1 ppm
Strömungsgeschwindigkeit: ~ 100 l/h

| Zeit [h] | 0 | 5 | 10 | 15 | 22 | 26 | 32 | 40,4 | 42,4 | 47,4 |
|---|---|---|---|---|---|---|---|---|---|---|
| Δf [Hz] | 0 | 156 | 212 | 260 | 312 | 354 | 394 | 464 | 480 | 532 |

| Zeit [h] | 50,4 | 52,4 | 57,4 | 60,4 | 62,4 | 65,4 | 70,4 | 72,4 | 80,4 | 84,4 |
|---|---|---|---|---|---|---|---|---|---|---|
| Δf [Hz] | 562 | 580 | 632 | 666 | 686 | 710 | 758 | 776 | 832 | 860 |

Die Beispiele zeigen, daß Ozon durch einen Sensor, der olefinische Seitengruppenpolymere enthält, nahezu linear nachgewiesen werden kann.

## Patentansprüche

1. Sensor aus einem piezoelektrischen Kristall mit einer ein olefinisches Seitengruppenpolymer enthaltenden Beschichtung.

2. Sensor nach Anspruch 1, **dadurch gekennzeichnet, daß** das olefinische Seitengruppenpolymer ein substituiertes Polyacrylat, Polymethacrylat, Polystyrol oder Polyester ist.

3. Sensor nach Anspruch 1 oder 2, **dadurch gekennzeichnet, daß** als olefinische Seitengruppenpolymere Copolymere mit einem Anteil von mindestens 3%, bevorzugt mindestens 15 % olefinischer Seitengruppen eingesetzt werden.

4. Sensor nach einem oder mehreren der Ansprüche 1 bis 3, **dadurch gekennzeichnet, daß** das mittlere Molekulargewicht im Bereich von 2 000 bis 10 000 000 g/mol, insbesondere von 10 000 bis 100 000 g/mol liegt.

5. Sensor nach einem oder mehreren der Ansprüche 1 bis 4, **dadurch gekennzeichnet, daß** der piezoelektrische Kristall eine Grundschwingung in einem Frequenzbereich von 20 kHz bis 100 MHz hat.

6. Sensor nach einem oder mehreren der Ansprüche 1 bis 5, **dadurch gekennzeichnet, daß** der piezoelektrische Kristall eine Oberschwingung oder eine surface acustic wave Schwingung im Frequenzbereich von 20 kHz bis 1000 MHz aufweist.

7. Sensor nach einem oder mehreren der Ansprüche 1 bis 6, **dadurch gekennzeichnet, daß** als piezoelektrischer Kristall ein Erdalkalititanat, Bleizirkoniumtitanat oder Quarz eingesetzt wird.

8. Sensor nach Anspruch 7, **dadurch gekennzeichnet, daß** als piezoelektrischer Kristall Quarz eingesetzt wird.

9. Verwendung eines Sensors nach Anspruch 1 zum Nachweis von Ozon.

## Claims

1. A sensor comprising a piezoelectric crystal having a coating comprising a polymer having olefinic side groups.

2. A sensor as claimed in claim 1, wherein the polymer having olefinic side groups is a substituted polyacrylate, polymethacrylate, polystyrene or polyester.

3. A sensor as claimed in claim 1 or 2, wherein the polymer having olefinic side groups which is used is a copolymer containing at least 3%, preferably at least 15%, of olefinic side groups.

4. A sensor as claimed in one or more of claims 1 to 3, wherein the mean molecular weight is in the range from 2 000 to 10 000 000 g/mol, in particular from 10 000 to 100 000 g/mol.

5. A sensor as claimed in one or more of claims 1 to 4, wherein the piezoelectric crystal has a fundamental vibration in a frequency range of from 20 kHz to 100 MHz.

6. A sensor as claimed in one or more of claims 1 to 5, wherein the piezoelectric crystal has a harmonic vibration or a surface acoustic wave vibration in the frequency range from 20 kHz to 1 000 MHz.

7. A sensor as claimed in one or more of claims 1 to 6, wherein the piezoelectric crystal used is an alkaline earth metal titanate, lead zirconate titanate or quartz.

8. A sensor as claimed in claim 7, wherein the piezoelectric crystal used is quartz.

9. Use of a sensor as claimed in claim 1 for detecting ozone.

**Revendications**

1. Sonde en cristal piézoélectrique présentant un recouvrement contenant un polymère d'oléfine à groupements latéraux.

2. Sonde selon la revendication 1, **caractérisée en ce que** le polymère d'oléfine à groupements latéraux est un polyacrylate substitué, un polyméthacrylate substitué, un polystyrène substitué ou un polyester substitué.

3. Sonde selon les revendications 1 ou 2, **caractérisée en ce qu'**on utilise comme polymères d'oléfine à groupements latéraux des copolymères présentant une proportion d'au moins 3%, de préférence d'au moins 15%, de groupements latéraux oléfiniques.

4. Sonde selon l'une ou plusieurs des revendications 1 à 3, **caractérisée en ce que** le poids moléculaire moyen est situé dans la plage de 2000 à 10000000 g/mole, en particulier de 10000 à 100000 g/mole.

5. Sonde selon l'une ou plusieurs des revendications 1 à 4, **caractérisée en ce que** le cristal piézoélectrique présente une oscillation de base dans une plage de fréquences de 20 kHz à 100 MHz.

6. Sonde selon l'une ou plusieurs des revendications 1 à 5, **caractérisée en ce que** le cristal piézoélectrique présente une oscillation harmonique ou une oscillation d'onde acoustique de surface (SAW - surface acoustic wave) dans la plage de fréquences de 20 kHz à 1000 MHz.

7. Sonde selon l'une ou plusieurs des revendications 1 à 6, **caractérisée en ce qu'**on utilise comme cristal piézoélectrique un titanate de métal alcalino-terreux, un titano-zirconate de plomb ou du quartz.

8. Sonde selon la revendication 7, **caractérisée en ce qu'**on utilise du quartz comme cristal piézoélectrique.

9. Utilisation d'une sonde selon la revendication 1 pour la détection d'ozone.